# EUROPEAN PATENT APPLICATION

(11) **EP 3 184 089 A1**
(43) Date of publication of application: **28.06.2017**
(21) Application number: 15201515.2
(22) Date of filing: 21.12.2015
(51) Int. Cl.: A61F 5/058, A61F 5/01

(54) **MOVEMENT RESTRAINING DEVICE**

(71) Applicant: 3M Innovative Properties Company, Saint Paul, MN 55133-3427 (US)
(72) Inventor: Graichen, Andreas Heinz, 40721 Hilden (DE); Gerullis, Michelle, 71364 Waiblingen-Beinstein (DE); Geerkens, Bernhard, 47877 Willich (DE); Bongards, Dr. Christine, 41516 Grevenbroich (DE)
(74) Representative: Müller, Bruno

(57) **Abstract**

Movement restraining device (1) for partially immobilizing a body joint (10). The device comprises stopper elements (50, 60) having an abutment surface (100) and an abutting surface (70). One stopper element is pivotable, relative to the other stopper element. The device further has skin attachment means (90, 120) for attaching the stopper elements to skin. The abutment surfaces are arranged or shaped such that, in an abutting pivot position, they prevent pivoting in a first pivot direction (150), while allowing pivoting in a second pivot direction (160), opposite to the first pivot direction.

## Description

The present invention relates to movement restraining devices in medical environments, for human patients as well as for animals.

Movement of body parts at joints needs to be restricted when the movement would impede healing or cause damage to the patient. For example, limbs of patients in (artificial) coma may be protected against uncontrolled movement when the patient is transported in order to protect his limbs and joints. Traditionally, a cast or a splint is applied at a body joint to prevent movement of a body part, when the joint is damaged or has recently been subject to surgery, for example. Similarly, when a catheter is inserted into a body part through skin into a blood vessel in the vicinity of a body joint, movement of the body part can create a kink in the catheter, which may lead to a loss of a blood pressure signal or to complete or partial blocking of a catheter tube. It is not unusual for a patient to make uncontrolled movements when waking up from anaesthesia after surgery or to get rid of inserted pieces as they are uncomfortable. Such movements of a body part can lead to kinking of a catheter or of catheter tubes, e.g. when a radialis arterial catheter is kinked by inward movements of the hand. Braces have frequently been used to completely block movement of the body part in order to avoid catheter kinking.

For some types of injuries or deformities it is not desirable to block movement of a body part at a joint completely. Generally, there is a trend towards early mobilization and partial immobilization to facilitate faster healing. An attempt towards only partial immobilization is described in U.S. patent no. 5'921'945, in which a splint body, fabricated of a resilient material, is disclosed. This splint body, however, presents some resistance to movements even in an "allowed" direction and allows movement, albeit against resistance, in "forbidden" directions. It appears desirable to provide a movement restraining device that presents no resistance to a movement in allowed directions and hard resistance to a movement in a forbidden direction.

The present invention attempts to address this need. It provides a movement restraining device for partially immobilizing a body joint facilitating rotation of a body part about a joint axis, the restraining device comprising
a) a first stopper element, comprising an abutment surface;
b) a second stopper element,
   - comprising an abutting surface;
   - being arranged, in an adjacency direction, adjacent to the first stopper element;
   - being pivotable, relative to the first stopper element, about a virtual pivot axis; and
c) skin attachment means for attaching the first and the second stopper element to skin at the body joint to be partially immobilized;
wherein the second stopper element can be pivoted into an abutting pivot position, in which position the abutting surface is in surface contact with the abutment surface, the surface contact preventing pivoting in a first pivot direction, while allowing pivoting in a second pivot direction, opposite to the first pivot direction.

The movement restraining device can be attached to the skin at a body joint via the skin attachment means. The restraining device and the body joint are thereby restricted to pivoting in the same pivot direction, by approximately the same angle and approximately parallel to each other. The limitations imposed by the abutment surface and the abutting surface on the pivoting of the second stopper element relative to the first stopper element cause a limitation of the rotation of the body part about the joint axis by an abutment. In the abutting pivot position, the surface contact of the abutting surface and the abutment surface prevent pivoting, i.e. further pivoting, of the second stopper element in the first pivot direction. On the other hand, the free pivoting of the restraining device from the abutting pivot position in the second pivot direction facilitates a free, unimpeded rotation of the body part about the joint axis in the second, opposite pivot direction. Partial immobilization of the joint is thus achieved by the restraining device, attached to the body joint, allowing free movement up to the abutting pivot position, and creating an abutment to block further movement beyond the abutting pivot position.

A movement restraining device according to the invention may be used to partially immobilize body joints like, for example, joints in a human wrist or elbow, a human ankle or knee, a human finger or toe. Similarly, it may be used for corresponding body joints of animals like horses, cats, dogs, or the like. Generally, a movement restraining device according to the invention provides particular benefits when used to partially immobilize body joints that facilitate rotation of a body part about a joint axis. In particular, the rotation of the body part may be a pivoting rotation of a body part. Hence the body joint may be a pivoting joint. The pivoting rotation of a body part may be a pivoting rotation of a body part with respect to another body part. An example of such a pivoting rotation is the pivoting rotation of the forearm about an elbow axis with respect to the upper arm.

The first stopper element may have the shape of a brick or of a cube or of a prism, specifically of a triangular prism. It may be made of, or comprise, a polymeric material. Alternatively, it may be made of, or comprise, for example, metal, wood, glass, or mineral material. The first stopper element may be formed as a single piece. It may comprise outer surfaces forming a hollow space between them.

The second stopper element may have the shape of a brick or of a cube. It may be made of, or comprise, a polymeric material. Alternatively, it may be made of, or comprise, for example, metal, wood, glass, or a mineral material. The second stopper element may be formed as a single piece. It may comprise outer surfaces forming a hollow space between them. Independent of its shape, a stopper element (the first, the second or any further stopper element) may be hollow or solid. It may be a single piece or comprise a plurality of pieces.

The first stopper element and the second stopper element may be adapted to be engaged with each other or may be engaged with each other. The first stopper element may comprise a recess for receiving a protrusion of the second stopper element. The second stopper element may comprise a protrusion for engagement with the first stopper element. Similarly, the second stopper element may comprise a recess for receiving a protrusion of the first stopper element. The first stopper element may comprise a protrusion for engagement with the second stopper element.

Alternatively, the first stopper element and the second stopper element may be not engaged with each other and/or may be not adapted to be engaged with each other.

An abutment surface, e.g. the abutment surface of the first stopper element, or an abutment surface of a third, fourth or of a further stopper element, may be an outer surface, or a portion of an outer surface, of the stopper element. An abutment surface may be flat or comprise a flat portion. Alternatively, an abutment surface may be curved or comprise a curved portion. A curved abutment surface may be curved in one direction, i.e. it may comprise a two-dimensional curvature. An example of a two-dimensional curvature is the curved surface of a cylinder. A curved abutment surface may be curved in two directions, i.e. it may comprise a three-dimensional curvature. An example of a three-dimensional curvature is the surface of a sphere.

The second stopper element, like any further stopper element, may also comprise an abutment surface, the "second abutment surface". Two or more or all abutment surfaces of stopper elements of a restraining device according to the invention, may be oriented, in the abutting pivot position, parallel to each other. They may be oriented parallel to each other within a +/-10-degree angle interval.

The abutment surface of the first stopper element and the abutting surface may be parallel and directly adjacent to each other in the abutting pivot position of the second stopper element.

An abutting surface, e.g. the abutting surface of the second stopper element, may comprise raised surface features that match with corresponding recessed surface features comprised in the abutment surface, when the second stopper element is in the abutting pivot position. This may help to define the abutting pivot position more precisely.

In the context of this disclosure, an abutting surface is understood to be in surface contact with an abutment surface if the two surfaces touch each other in more than one point or along more than one line, i.e. when there is an extended contact area, or in other words, a surface contact, between the two surfaces. For the avoidance of doubt, two flat surfaces are not considered to be in contact when they are not parallel to each other, even though they might have a common edge.

The second stopper elements is arranged adjacent to the first stopper element, in an adjacency direction. The adjacency direction is defined by the arrangement of the second stopper element relative to the first stopper element. An adjacency direction may be the direction of a vector connecting a point on a surface of the first stopper element with a point on a surface of the second stopper element.

If the first and the second stopper element have identical outer shapes, a vector from the geometric centre of the first stopper element to the geometric centre of the second stopper element defines the adjacency direction. If the first and the second stopper element have identical outer shapes, a vector from a specific feature of the first stopper element to the corresponding specific feature of the second stopper element may define the adjacency direction. Generally, where two or more stopper elements are arranged adjacent to each other in a straight line or chain, the direction of this line is an adjacency direction. Most generally, an adjacency direction may be defined by a line connecting any point in the first stopper element to any point in the second stopper element.

Two stopper elements of a movement restraining device are considered adjacent stopper elements if they are arranged next to each other such that no other stopper element is arranged between them. However, adjacent stopper elements may be separated by a gap. They may be separated by a gap when the second stopper element is in a pivot position different from the abutting pivot position.

The gap may be narrow. It may, for example, be smaller than 1 mm, 2mm, or 5mm, as measured in an adjacency direction.

The second stopper element is pivotable relative to the first stopper element about a virtual pivot axis. The pivot axis may extend perpendicularly to the adjacency direction. The term "pivotable", in the context of the present disclosure, implies rotation about a single axis. A first pivot direction may be an angular direction of rotation about the pivot axis, e.g. clockwise, when viewed from a certain point. An opposite pivot direction may then be the opposite angular direction of rotation about the same axis, e.g. anticlockwise, when viewed from the same viewpoint. The second stopper element is pivotable between at least the abutting pivot position and a non-abutting pivot position. The second stopper element can be brought, i.e. pivoted, into the abutting pivot position, i.e. it can be brought, i.e. pivoted, from a non-abutting pivot position into the abutting pivot position.

The pivot axis is a mathematical, i.e. virtual, line about which the second stopper element can rotate or pivot. The pivot axis, in particular its position and orientation, may be defined by a physical element, e.g. by a hinge axis or a pin. The virtual pivot axis may be defined, for example, by a recess comprised in the first stopper element. The recess may be adapted to receive, or may receive, a matching protrusion comprised in the second stopper element, so that the second stopper element may be pivotably engaged with the first stopper element.

The pivot axis may be defined by a flexible adhesive tape connecting the first stopper element and the second stopper element. A first portion of the tape may be attached to the first stopper element, and a second portion of the tape may be applied to the second stopper element. A third portion, or generally a portion, of the tape may form a hinge and a hinge axis or pivot axis, about which the second stopper element is pivotable relative to the first stopper element. Therefore, more generally, the pivot axis may be defined by the skin attachment means or a portion of the skin attachment means.

The pivot axis may be defined by an axis of a hinge arranged between the first stopper element and the second stopper element.

Alternatively, the pivot axis may extend in a direction parallel to the adjacency direction.

Where the movement restraining device comprises a third stopper element, the third stopper element may be arranged pivotably relative to the second stopper element as described above for the second stopper element. In particular, a virtual pivot axis of the third stopper element may be arranged parallel to the virtual pivot axis of the second stopper element. This allows the second and the third stopper elements to pivot in the same pivot direction, which is advantageous in that it allows movement of the joint, to which the restraining device is attached, in the pivot direction.

Similarly, the third stopper element may be arranged pivotably relative to the first stopper element as described above for the second stopper element. In particular, a virtual pivot axis of the third stopper element such arranged may be arranged parallel to the virtual pivot axis of the second stopper element. This allows the second and the third stopper elements to pivot in the same pivot direction, which is advantageous in that it allows movement of the joint, to which the restraining device is attached, in the pivot direction.

Generally, for a restraining device comprising a third stopper element and further stopper elements, each adjacent to another stopper element in the adjacency direction of the second stopper element, these stopper elements may be pivotable such that the respective pivot axes of all these stopper elements are oriented parallel to the pivot axis of the second stopper element.

The movement restraining device of the present disclosure comprises skin attachment means for attaching the first and second stopper elements to the skin at the body joint which is to be partially immobilized. The word skin is to be understood, in the context of this disclosure, to describe the outer surface of the body, i.e. it encompasses bare skin, hairy skin, furred skin, and any skin of humans and animals.

Generally, stopper elements can be attached to skin in various ways. Skin attachment means may thus, for example, comprise a belt member for pulling around the stopper element and the body part to which the stopper element is to be attached. Other, alternative attachment means include, for example, vacuum methods, mechanical fasteners, hook and loop type straps, and sutures.

In a movement restraining device according to the present invention, the first stopper element may have the shape, e.g. the overall shape, of a parallelepiped. The second stopper element may have the shape, e.g. the overall shape, of a parallelepiped. Parallelepipeds comprise brick shapes and cube shapes and other shapes. Specifically, the first stopper element and the second stopper element may have the shape of a parallelepiped. In devices according to the invention comprising first, second and further stopper elements, all stopper elements may have the shape of parallelepipeds. All stopper elements may have the shape of a parallelepiped, i.e. of the same parallelepiped. Parallelepiped shapes may be advantageous in that they comprise six surfaces, of which one can be used as an abutment surface, one can be used as abutting surface, and one can be used as a skin-facing surface. Also, parallelepiped-shaped stopper elements may have a high stiffness, because the surfaces support each other mechanically, so that deformation requires rather high forces.

Specifically, the first stopper element and/or the second stopper element, or generally all stopper elements, may have the shape of bricks. A brick shape may be advantageous in that it provides surfaces that are orthogonal to each other. Where the second stopper element comprises a second skin-facing surface, this in turn may allow the abutting surface to be oriented perpendicular to the second skin facing surface, which may provide for a particularly strong abutment, in certain geometries.

The first stopper element and the second stopper element may have identical outer shapes. Generally, all stopper elements of a movement restraining device according to the present invention, may have identical outer shapes. Movement restraining devices made from identically shaped stopper elements may bring along cost benefits in manufacturing.

The first stopper element may comprise a first skin-facing surface. The second stopper element may comprise a second skin-facing surface. The skin attachment means may be adapted for attaching the first and the second stopper element to skin at the body joint in a way that the first and the second skin-facing surfaces are oriented towards the skin. Dedicated skin-facing surfaces may be advantageous in that they may allow more reliable adhesion to the skin, e.g. by an adhesive. They may also better define orientation of the stopper elements relative to the skin surface.

A skin-facing surface, i.e. the first and/or the second skin-facing surface, and/or a skin-facing surface of a third, fourth and/or of a further stopper element, may be an outer surface, or a portion of an outer surface, of the stopper element. A skin-facing surface may be flat or comprise a flat portion. Alternatively, a skin-facing surface may be curved or comprise a curved portion. A curved skin-facing surface may be curved in one direction, i.e. it may comprise a two-dimensional curvature. A curved skin-facing surface may be curved in two directions, i.e. it may comprise a three-dimensional curvature. A curved skin-facing surface may be shaped to follow the contour of the skin at the body joint, so that the restraining device may be more comfortable to wear and/or it may be easier to attach the stopper element to the skin at the body joint.

Where the stopper elements have skin-facing surfaces, the pivot axis may be defined by a flexible adhesive tape connecting the first stopper element and the second stopper element. A first portion of the tape may be attached to the first skin-facing surface, a second portion of the tape may be applied to the second skin-facing surface. A third portion, or generally a portion, of the tape may form a hinge and a hinge axis, about which the second stopper element is pivotable relative to the first stopper element.

The virtual pivot axis may extend perpendicularly to the adjacency direction. Where the first stopper element has a rectangular side face, i.e. a rectangle-shaped surface portion facing the second stopper element, the pivot axis may extend parallel to an edge of the rectangle. Where a first edge of the side face rectangle extends parallel to the first skin-facing surface, the pivot axis may extend parallel to the first edge. This arrangement may allow for pivoting the second stopper element away from or towards the skin.

Alternatively, where a second edge of the side face rectangle extends perpendicular to the first skin-facing surface, the pivot axis may extend parallel to the second edge. This arrangement may allow for pivoting the second stopper element parallel to the skin.

The abutment surface of the first stopper element may be flat. Where the first stopper element comprises a first skin-facing surface, the abutment surface of the first stopper element may be oriented perpendicularly to the first skin-facing surface. Similarly, the abutting surface of the second stopper element may be flat. Where the second stopper element comprises a second skin-facing surface, the abutting surface of the second stopper element may be oriented perpendicularly to the second skin-facing surface. In a specific aspect of the invention, both the abutment surface may be flat and oriented perpendicularly to the first skin-facing surface, and the abutting surface of the second stopper element may be oriented perpendicularly to the second skin-facing surface.

Such geometries may result in generating less force in a direction that would lift the device off the skin, when the stopper elements are in the abutting pivot position.

The abutment surface may be flat. Any skin facing surface, e.g. the first or the second skin-facing surface, may be flat. The surface normal of the abutment surface may be oriented at an angle of between 60° and 120° with respect to the surface normal of the first skin-facing surface. For movement restraining devices having more than two stopper elements, such a geometry may provide for particularly small bending radius of the movement restraining device in a particular portion of the device, which may be desirable for use at small body joints requiring a small bending radius.

The abutment surface may be flat and oriented parallel to the first skin-facing surface. Such a geometry may provide for a particularly thin movement restraining device, i.e. one that extends less in a direction away from the skin, e.g. when the stopper elements are in the abutting pivot position.

The abutting surface may be flat and oriented parallel to the second skin-facing surface. This geometry may provide for a particularly thin movement restraining device, i.e. one that extends less in a direction away from the skin, e.g. when the stopper elements are in the abutting pivot position.

In the abutting pivot position, the abutment surface and the abutting surface may be parallel to each other within an angle of +/- 10°. Parallel surfaces can form an effective abutment, avoid stress concentration in contact points, and potentially transmit higher forces.

In a movement restraining device according to the present disclosure, in which the first stopper element comprises a first skin-facing surface, the pivot axis may be oriented parallel to the first skin-facing surface. This orientation of the pivot axis may allow for pivoting of the second stopper element, relative to the first stopper element, such that its skin-facing surface (the second skin-facing surface) pivots out of the plane of the first skin-facing surface. This may make the device particularly well suited for application to the inner bend of a hinge-type joint, e.g. inside an elbow joint.

Alternatively, the pivot axis may be oriented perpendicular to the first skin-facing surface. This orientation of the pivot axis may allow for pivoting of the second stopper element, relative to the first stopper element, such that its skin-facing surface (the second skin-facing surface) pivots "sideways" in the plane of the first skin-facing surface. This may make the device particularly well suited for application to a lateral side of a hinge-type joint, e.g. at the side of an elbow joint. The skin attachment means may comprise an adhesive, for example a pressure-sensitive adhesive. The adhesive may be arranged on an outer surface of the stopper element. In particular, it may be arranged on a skin-facing surface, on the first skin-facing surface and/or on the second skin-facing surface or on the skin-facing surfaces of all stopper elements of the restraining device. Specifically, the skin attachment means may comprise a pressure-sensitive adhesive arranged on at least a portion of a skin-facing surface.

More specifically, the skin attachment means may comprise a layer of adhesive, e.g. a layer of pressure-sensitive adhesive. The layer of adhesive may be arranged on an outer surface of the stopper element. In particular, it may be arranged on a skin-facing surface, on the first skin-facing surface and/or on the second skin-facing surface, or on the skin-facing surfaces of all stopper elements of the restraining device. Specifically, the skin attachment means may comprise a layer of pressure-sensitive adhesive arranged on at least a portion of a skin-facing surface. The layer of adhesive may be continuous or patterned.

Generally, attachment of a movement restraining device to skin via adhesive may provide for attachment over an extended area of the skin, thus distributing mechanical stress over a large skin area. This may avoid injuries or bruises on the skin. Also, attachment via adhesive may be a space-saving way of attaching a movement restraining device to the body of a patient or an animal. Adhesive attachment may also be a cost-effective means of attachment, in particular for one-time, single-use movement restraining devices according to the present disclosure.

Generally, the adhesive may be a skin-friendly adhesive.

The skin attachment means in a device according to the present invention may comprise a carrier tape. Such a carrier tape has a first and an opposed second major surface. It may comprise a polymeric material or a woven or a nonwoven fabric. The skin attachment means may comprise a carrier tape and pressure-sensitive adhesive, arranged on one major surface of the carrier tape. The pressure-sensitive adhesive may be suitable for attaching the carrier tape to the skin.

The skin attachment means may comprise a carrier tape having two opposed major surfaces, wherein the layer of adhesive is arranged on one of the major surfaces, e.g. on the first major surface of the carrier tape. The first stopper element may then be attached to the second, opposed major surface of the carrier tape. Specifically, the first skin-facing surface of the first stopper element may be attached to the second, opposed major surface of the carrier tape. The first stopper element can thereby be attached to the skin via the carrier tape and the adhesive.

Alternatively, the first and the second stopper element may be attached to the second, opposed major surface of the carrier tape. The first skin-facing surface of the first stopper element and the second skin-facing surface of the second stopper element may be attached to the second, opposed major surface of the carrier tape. Generally, for movement restraining devices having first, second and further stopper elements comprising skin-facing surfaces, the skin-facing surfaces of all stopper elements may be attached to the second major surface of the carrier tape.

The carrier tape may be flexible or bendable. A flexible or bendable carrier tape may provide for a connection between the first stopper element and the second stopper element. Specifically, a bendable or flexible carrier tape may provide for a pivotable connection between the stopper elements.

The skin attachment means may comprise a first and a second carrier tape. The first stopper element may be attached to skin via the first carrier tape. The second stopper element may be attached to skin via the second carrier tape. Specifically, the first skin-facing surface of the first stopper element may be attached to skin via the first carrier tape. The second skin-facing surface of the second stopper element may be attached to skin via the second carrier tape.

The first stopper element and the second stopper element may be attached to each other by the skin attachment means. Where the skin attachment means comprises a carrier tape, they may be attached to each other by the carrier tape. The stopper elements may be attached to each other such that the second stopper element is pivotable with respect to the first stopper element. Generally, all stopper elements may be attached to respective adjacent stopper elements by the skin attachment means.

Such an attachment may provide for all components of the movement restraining device being connected with each other, which may facilitate handling of the device or its application to the skin or both.

A movement restraining device according to the present disclosure may comprise a plurality of further stopper elements, each comprising an abutment surface, an abutting surface, and a skin-facing surface. Each of the further stopper elements may be arranged, in the adjacency direction, adjacent to the first stopper element or to the second stopper element or to one of the further stopper elements. Each further stopper element may be pivotable, relative to an adjacent stopper element, about a virtual pivot axis. The pivot axis may, for example, extend perpendicularly to the adjacency direction. Each of the further stopper elements may comprise skin attachment means for attaching the further stopper element to skin at the body joint to be partially immobilized in a way, that the skin-facing surface of the further stopper element is oriented towards the skin. The abutment surface of each further stopper element may be arranged and/or shaped such that, in an abutting pivot position, the abutment surface of the further stopper element is in contact with the abutting surface of the adjacent stopper element, thereby preventing pivoting in a first pivot direction, while allowing pivoting in a second pivot direction, opposite to the first pivot direction.

A movement restraining device as described here may thus comprise a chain of stopper elements, each adjacent to an adjacent stopper element in the adjacency direction as defined by the arrangement of the first and the second stopper elements. Each of the further stopper elements is pivotable with respect to its neighbour, so that the chain is deformable and can change its form, e.g. from straight to curved, thereby following the movement of the body joint at which the device is attached to skin. The individual stopper elements can be pivoted relative to their neighbour up to individual abutting pivot positions, defined by position, shape and orientation of the abutment surface of one stopper element relative to the abutting surface of the adjacent stopper element. Thereby the change of the form of the entire chain of stopper elements is limited. The movement restraining device as a whole thereby provides an abutment position, which results from the combined effect of the abutting pivot positions of the individual stopper elements with respect to their adjacent stopper elements. Because the entire device is attached to the skin at the body joint, the abutment position of the entire device defines an abutment for the rotation of a body part rotating about the axis of the body joint.

For a given length of the movement restraining device, i.e. its extension in the adjacency direction, more but smaller stopper elements may allow for the device to follow a body contour more closely at the body joint. This may benefit adhesion and comfort of the patient.

Generally, partial immobilization of a smaller body joint, e.g. of a finger joint, may require smaller stopper elements, because there is little space to attach stopper elements to skin. However, more reliable attachment of the movement restraining device to skin may generally require a larger surface of the skin attachment means, which may be achieved by having larger stopper elements, and in particular larger skin-facing surfaces of the stopper elements. Skin attachment means may therefore extend beyond the skin-facing surface of the first or the second stopper element. Generally, the skin attachment means may extend beyond the skin-facing surfaces of all stopper elements. This may help make the attachment of stopper elements to skin more reliable.

The first stopper element, the second stopper element and any and all of the further stopper elements may be a single piece. In particular, the skin-facing surface, the abutting surface and the abutment surface of any stopper element may be surfaces of a single piece. "Single piece" is to be understood, in the context of this disclosure, as having the sense of "made from a single piece of material", as opposed to elements that are assembled from two or more components.

A movement restraining device as described above will often be used to restrain movement of a human wrist joint, such that a radialis catheter inserted into the artery at the wrist is prevented from kinking, e.g. when the patient bends his hand inward inadvertently after surgery and anaesthesia. Any tubes or lumen connected to the catheter can advantageously be guided or routed by the movement restraining device, which is anyway arranged at the wrist and hence close to the catheter. A tube organizer comprised in one of the stopper elements may help keep the tubes in their position and prevent dislodgement of the catheter through pull force on a tube. It may help prevent kinking of the tubes. It may also save space in the vicinity of the catheter, because no separate tube organizer is required. Also, it may provide for a more orderly arrangement and guiding of tubes and lumen, which in turn may help identify a specific lumen quicker and more reliably.

Hence, in a movement restraining device as described above, the first stopper element or the second stopper element or a further stopper element may comprise a tube organizer for routing and/or fixation of catheter tubes or lumen.

The tube organizer may comprise a groove for receiving a section of a tube or lumen. The groove may be shaped such as to bring the tube or lumen received in the groove into a straight or into a curved shape. A curved shape may have a suitable curvature such as to create friction between inner walls of the groove and the tube or lumen. The amount of friction created may be sufficient to prevent axial displacement of the tube or lumen under axial pull forces, e.g. under axial pull forces of up to 10 Newton on the tube or lumen. The length direction of a tube defines axial directions.

Independent of any other features, a movement restraining device according to the present disclosure may further comprise a first electrical contact element and a second electrical contact element, at least one of which is connectable to an indicator device for providing a visual, audible or electronic indication when the contact elements are in electrical contact with each other, wherein the first contact element can be brought into electrical contact with the second contact element by pivoting the second stopper element into the abutting pivot position.

The electrical contacts may facilitate detection and indication of the second stopper element being pivoted into the abutting pivot position. An indicator device may provide an indication, e.g. an audible, visible or electronic indication or alarm, to a nurse, a health worker, or to the patient himself that the partially-immobilized body part has been brought into a limit position, corresponding to the abutting pivot position of the second stopper element. This, in turn, may allow to take appropriate measures for protecting the patient.

The present disclosure also provides a method of partially immobilizing a body joint facilitating rotation of a body part about a body joint axis, the method comprising the steps of
a) providing a movement restraining device as described above; and
b) attaching the first stopper element and the second stopper element to skin at the body joint such that the pivot axis is oriented parallel to the body joint axis.

These steps may be performed in this sequence.

The invention will now be described in more detail with reference to the following

Figures exemplifying particular embodiments of the invention. Certain dimensions may be shown exaggerated for greater clarity.
- Fig. 1: Side view of a first movement restraining device according to the invention;
- Fig. 2: Side view of the first movement restraining device in an abutting pivot position;
- Fig. 3: Perspective view of a second movement restraining device according to the invention, comprising a carrier tape;
- Fig. 4: Perspective view of a third movement restraining device according to the invention, applied to a human wrist, in a non-abutting pivot position;
- Fig. 5: Perspective view of the third movement restraining device in an abutting pivot position;
- Fig. 6: Perspective view of a fourth movement restraining device according to the invention, with an extended carrier tape;
- Fig. 7: Side view of a fifth movement restraining device according to the invention, having step-shaped stopper elements, in a non-abutting pivot position;
- Fig. 8: Side view of a the fifth movement restraining device in an abutting pivot position;
- Fig. 9: Side view of a sixth movement restraining device according to the invention, having L-shaped stopper elements, in a non-abutting pivot position;
- Fig. 10: Side view of the sixth movement restraining device in an abutting pivot position;
- Fig. 11: Side view of a seventh movement restraining device according to the invention, in a non-abutting pivot position;
- Fig. 12: Side view of the seventh movement restraining device, in an abutting pivot position;
- Fig. 13: Perspective view of a first and a second stopper element usable for a movement restraining device according to the invention;
- Fig. 14: Perspective view of an eighth movement restraining device according to the invention, comprising the stopper elements shown in Fig. 13, in an abutting pivot position;
- Fig. 15: Perspective view of a stopper element usable for a movement restraining device according to the invention; and
- Fig. 16: Perspective view of three further stopper elements usable for movement restraining devices according to the invention.

Herein below various embodiments of the present invention are described and shown in the drawings wherein like elements are provided with the same reference numbers.

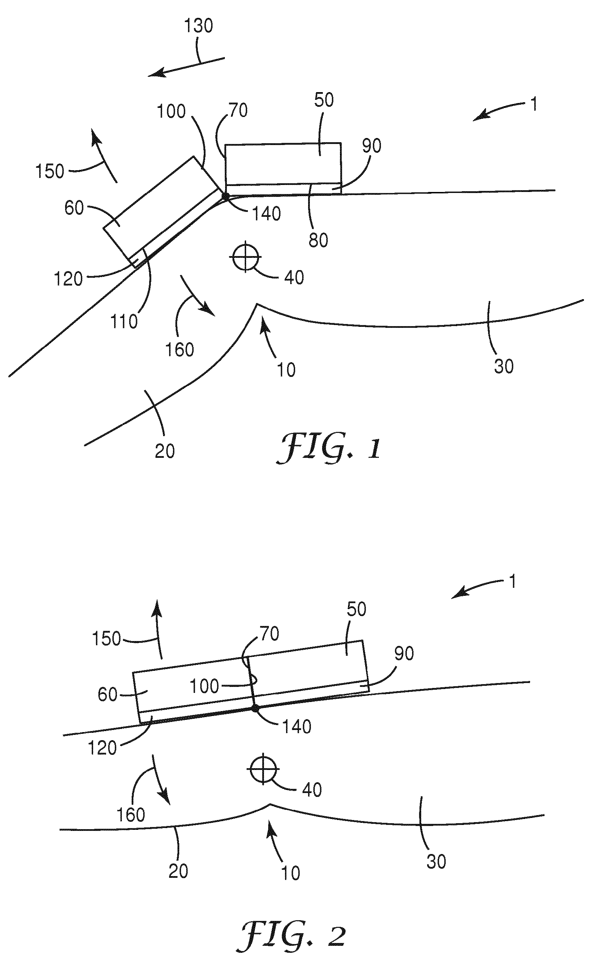

**Figure 1** is an idealized side view of a first movement restraining device 1, attached to skin at a body joint 10 of a human patient. The body joint 10 facilitates rotation of a first body part 20, relative to a second body part 30, about a joint axis 40, which is oriented perpendicularly to the plane of the drawing.

The movement restraining device 1 comprises a first stopper element 50 and a second stopper element 60. Both stopper elements 50, 60 have identical outer shapes, namely the shape of a brick, of which a side surface is visible in the Figure. The first stopper element 50 comprises a flat abutment surface 70 and a first skin-facing surface 80. The abutment surface 70 is oriented perpendicularly to the first skin-facing surface 80, and in the plane of the drawing their surface normals form an angle of 90° relative to each other. The first stopper element 50 is attached to the skin via a first adhesive layer 90, which is arranged on the first skin-facing surface 80. The first adhesive layer 90 is a layer of a skin-friendly pressure-sensitive adhesive.

The second stopper element 60 comprises an abutting surface 100 and a second skin-facing surface 110. The second stopper element 60 is attached to the skin via a second adhesive layer 120, arranged on the second skin-facing surface 110. The second stopper element 60 is arranged adjacent to the first stopper element 50. The adjacency direction is indicated by arrow 130. It extends in the plane of the drawing. The adjacency direction 130 indicates a direction which points generally from the first stopper element 50 towards the second stopper element 60.

The second stopper element 60 is pivotable relative to the first stopper element 50, about a virtual pivot axis 140, which extends perpendicularly to the plane of the drawing and parallel to the first skin-facing surface 80. The virtual pivot axis 140 is perpendicular, i.e. orthogonal, to the adjacency direction 130.

In Figure 1, the movement restraining device 1 is shown in a pivot position which is not an abutting pivot position. In the pivot position shown in Figure 1, the first body part 20 can be freely rotated, relative to the second body part 30, about the joint axis 40 in a first pivot direction, indicated by arrow 150, i.e. clockwise, or in a second pivot direction, indicated by arrow 160, opposite to the first pivot direction 150, i.e. anti-clockwise (in the drawing). In the pivot position shown in Figure 1, the movement of the body joint 10 is not impeded. Because the movement restraining device 1 is attached to the skin and thus pivots about the pivot axis 140 as the first body part 20 rotates about the joint axis 40, the anti-clockwise rotation of the first body part 20 is limited by the abutting surface 100 of the second stopper element 60 abutting against the abutment surface 70 of the first stopper element 50 in an abutting pivot position. This is shown in Figure 2.

**Figure 2** is an idealized side view of the first movement restraining device 1, attached to skin at the body joint 10. The device 1 is shown in the abutting pivot position, in which the abutting surface 100 of the second stopper element 60 is in surface contact with the abutment surface 70 of the first stopper element 50.

The abutment of the abutting surface 100 against the abutment surface 70 prevents pivoting, or further pivoting, in the first pivot direction 150. Due to the device 1 being attached to skin, the movement restraining device 1 prevents rotation, i.e. further rotation, of the first body part 20 about the joint axis 40 in clockwise direction (in the drawing) beyond the position shown in Figure 2.

This mechanism provides partial immobilization of the body joint 10. The immobilization is partial, because the first body part 20 can be rotated freely in the anti-clockwise direction, while it can rotate in a clockwise direction only up to the abutment provided by the contact of the abutment surface 70 and the abutting surface 100 in the abutting pivot position. This helps avoid overstretching of the body joint 10 beyond the almost-straight position shown in Figure 2.

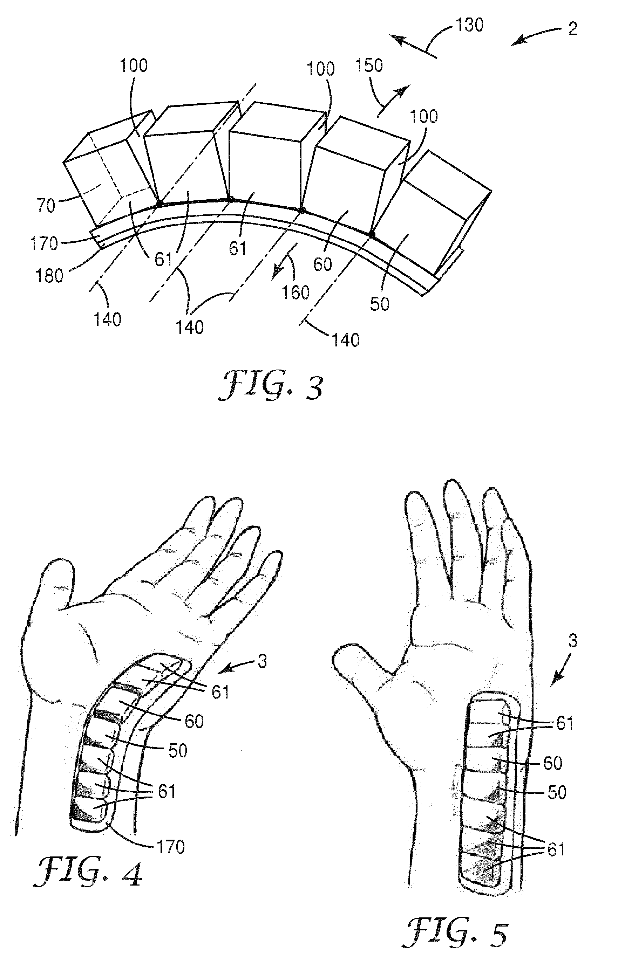

A movement restraining device according to the present disclosure may comprise more than two stopper elements 50, 60. An example of such a movement restraining device is shown in **Figure 3****,** in which a second movement restraining device 2 is shown in perspective view. It comprises, in addition to a first stopper element 50 and a second stopper element 60, three further stopper elements 61. Each of the stopper elements 50, 60, 61 has the shape of a cube and comprises an abutment surface 70 and an abutting surface 100, and a skin-facing surface. The skin-facing surfaces are oriented towards the bottom in the Figure, and are therefore not visible in Figure 3). The stopper elements 50, 60, 61 are arranged adjacent to a neighbouring stopper element 50, 60, 61 in an adjacency direction 130. In other words, the stopper elements 50, 60, 61 form a chain of stopper elements, one adjacent to another in the adjacency direction 130 defined by the arrangement of the first stopper element 50 and the second stopper element 60.

Each of the stopper elements 50, 60, 61 is pivotable, relative to a neighbouring stopper element 50, 60, 61 about a virtual pivot axis 140. The pivot axes 140 are parallel to each other and extend perpendicularly to the adjacency direction 130.

The device 2 comprises skin attachment means in the form of a flexible carrier tape 170 and an adhesive layer 180. The carrier tape 170 and the adhesive layer 180 can be bent such as to allow pivoting of the stopper elements 50, 60, 61 relative to their neighbouring stopper elements 50, 60, 61. The carrier tape 170 is a continuous polymeric film. It is adhesively attached to the skin-facing surfaces of the stopper elements 50, 60, 61. The stopper elements 50, 60, 61 are thus attached to each other by the carrier tape 170. On the underside (in Figure 3) of the carrier tape 170 the adhesive layer 180 is arranged, through which the stopper elements 50, 60, 61 of the movement restraining device 2 can be attached to the skin such that their respective skin-facing surface faces the skin.

The abutment surfaces 70 are arranged and shaped such that, in an abutting pivot position, the abutment surface 70 of a stopper element 50, 60, 61 is in contact with the abutting surface 100 of the adjacent stopper element 50, 60, 61, so that further pivoting in a first pivot direction 150 is prevented, while pivoting in the second pivot direction 160, opposite to the first pivot direction 150, is still possible.

For each pair of stopper element 50, 60, 61 and adjacent stopper element 50, 60, 61, a pivot axis 140 can be defined, about which one stopper element 50, 60, 61 can pivot relative to the adjacent stopper element 50, 60, 61. Similarly, each pair of stopper element and adjacent stopper element define, through their relative arrangement, orientation and through the shape of the abutting surface 100 and abutment surface 70, an individual abutting pivot position. Once attached to the skin at a body joint, the movement restraining device 2 limits the movement of the body joint when all pairs of adjacent stopper elements 50, 60, 61 are in their abutting pivot position.

**Figures 4 and 5** show, in a perspective view, how a movement restraining device according to the present disclosure can be applied to a human wrist. The wrist may not bend inwards, because an inward bend (i.e. inner hand towards forearm and shoulder) may cause a catheter (not shown), inserted into the radialis artery, to kink and to block the flow of liquid or gas through it. The movement restraining device 3 of Figure 4 comprises a first stopper element 50, a second stopper element 60, and five further stopper elements 61. Apart from the shape of the stopper elements 50, 60, 61, and the size of the carrier tape 170, the movement restraining device 3 is identical to the device 2 shown in Figure 3.

The stopper elements 50, 60, 61 are attached to the skin at the wrist joint by a single carrier tape 170 and an adhesive layer underneath the carrier tape (not visible in Figure 4). The carrier tape 170 and the adhesive layer extend further than the stopper elements 50, 60, 61. Thereby they provide a larger adhesive surface for attachment to the skin, which results in a more reliable attachment of the movement restraining device 3 to the skin.

The movement restraining device 3 is shown in a position, in which not all pairs of adjacent stopper elements 50, 60, 61 are in an abutting pivot position with respect to each other. The movement restraining device 3 allows movement of the wrist joint such that the upper side of the hand can be bent towards the shoulder and forearm, as is shown in Figure 4. It prevents, however, an inward bending of the hand. This is shown in Figure 5, where all pairs of adjacent stopper elements 50, 60, 61 are in an abutting pivot position with respect to each other. In this position, the abutment surfaces 70 and the abutting surfaces 100 of all pairs of adjacent stopper elements 50, 60, 61 are in contact with each other, and thereby prevent any further pivoting of any one of the stopper elements 50, 60, 61 in the first pivot direction, i.e. in a direction where the hand would bend inward relative to the forearm.

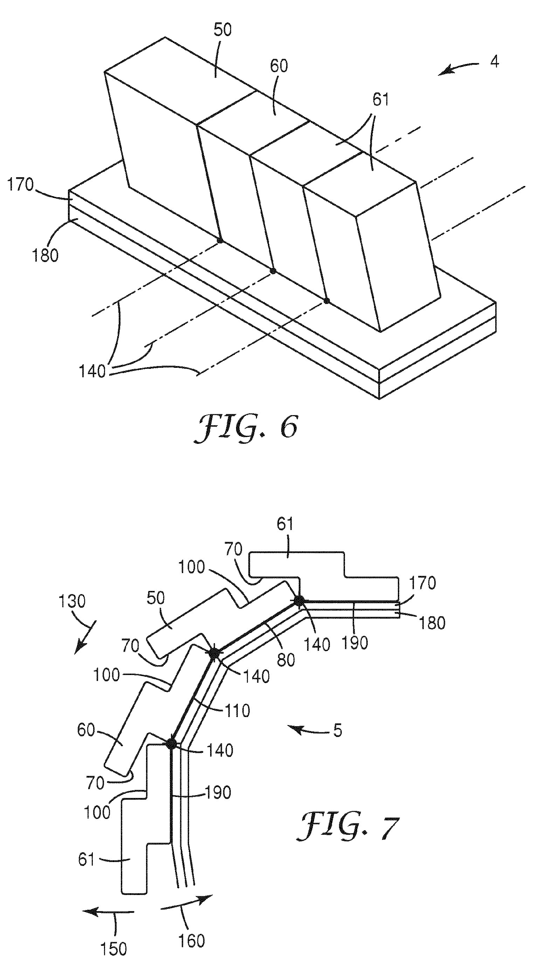

**Figure 6** is a sketched perspective view of a fourth movement restraining device 4. It is similar to the third movement restraining device 3, except for the number and shape of the stopper elements 50, 60, 61. The stopper elements 50, 60, 61 are shaped as parallelepipeds, i.e. deformed bricks in which not all angles are right angles. The first stopper element 50 is about twice as large as the second stopper element 60, while the further stopper elements 61 have the same size as the second stopper element 60. All stopper elements 50, 60, 61 are attached with their skin-facing surfaces (not visible in this Figure) to a flexible carrier tape 170. The flexible carrier tape 170 and the adhesive layer 180 on its underside extend beyond the skin-facing surfaces of the stopper elements 50, 60, 61. The movement restraining device is shown in a position, in which all pairs of adjacent stopper elements 50, 60, 61 are in an abutting pivot position. For greater clarity, the pivot axes 140 are drawn, about which a stopper element 60, 61 can pivot relative to an adjacent stopper element 50, 60, 61.

Stopper elements of a movement restraining device according to the present invention can have different shapes and sizes. **Figure 7** is a side view of a fifth movement restraining device 5, in which the stopper elements 50, 60, 61 have a stepped shape, and a stepped cross section in a plane orthogonal to the adjacency direction 130 and orthogonal to the first skin-facing surface 80.

The first stopper element 50 comprises a first skin-facing surface 80 and a flat abutment surface 70, which is oriented parallel to the first skin-facing surface 80.

The second stopper element 60 comprises a second skin-facing surface 110 and a flat abutting surface 100. The abutting surface 100 is parallel to the second skin-facing surface 110. The second stopper element 60 is arranged adjacent to the first stopper element 50 in an adjacency direction 130, which is in the plane of the drawing. It is pivotable relative to the first stopper element 50, about a pivot axis 140 which is orthogonal to the adjacency direction 130. The pivot axis 140 extends perpendicular to the plane of the drawing.

The device 5 can be attached to skin at a body joint via a flexible carrier tape 170, to which the skin-facing surfaces 80, 110, 190 of the first stopper element 50, the second stopper element 60 and further stopper elements 61 are attached. The carrier tape 170 carries, on its skin-facing major surface, an adhesive layer 180, through which the stopper elements 50, 60, 61 can be attached to skin such that their skin-facing surfaces 80, 110, 190 are oriented towards the skin.

The second stopper element 60 and the further stopper elements 61 have outer shapes that are identical to the outer shape of the first stopper element 50. All stopper elements 50, 60, 61 comprise an abutment surface 70 and an abutting surface 100.

The carrier tape 170 is flexible enough to allow pivoting of the second stopper element 60 relative to the first stopper element 50, and generally of a stopper element 50, 60, 61 relative to a neighbouring, adjacent stopper element 50, 60, 61.

The first stopper element 50 and the second stopper element 60, and all further stopper elements 61, are attached to each other by the carrier tape 170. The carrier tape 170 provides the virtual pivot axes 140 for pivoting a stopper element 50, 60, 61 with respect to an adjacent stopper element 50, 60, 61.

In Figure 7, the fifth movement restraining device 5 device is shown in a state in which the first stopper element 50 and the second stopper element 60 are not in an abutting pivot position. Further, none of the stopper elements 50, 60, 61 is in an abutting pivot position relative to an adjacent stopper element 50, 60, 61. In this position, pivoting of the second stopper element 60 and of any further stopper element 60, 61 in the first pivot direction 150 and in the second, opposed pivot direction 160 is not impeded.

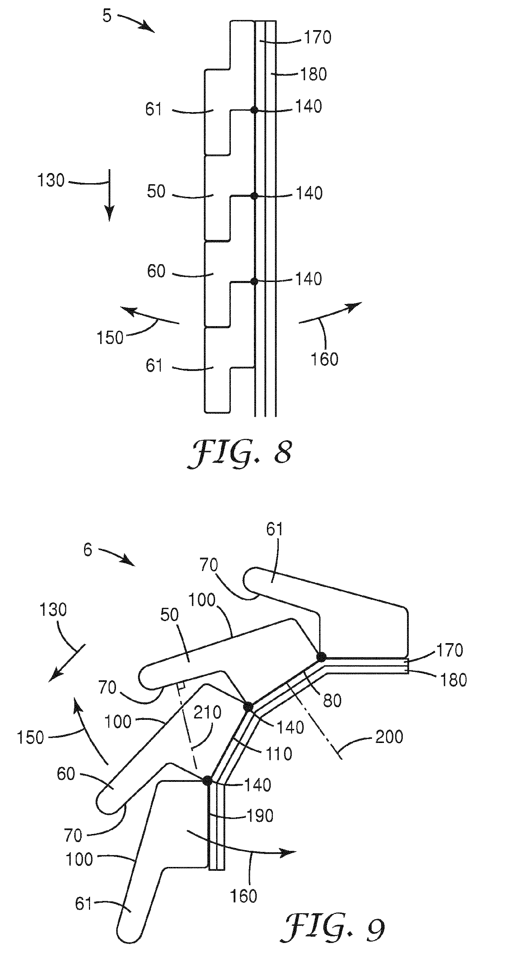

**Figure 8** shows, in side view, the fifth movement restraining device 5 in a state, in which the second stopper element 60 is in an abutting pivot position with respect to the first stopper element 50. Further, all of the stopper elements 50, 60, 61 are in an abutting pivot position relative to an adjacent stopper element 50, 60, 61. In this position, further pivoting of the second stopper element 60 and of any further stopper element 60, 61 in the first pivot direction 150 is impossible, while pivoting in the second pivot direction 160 is not impeded.

**Figure 9** is a side view of a sixth movement restraining device 6 comprising four stopper elements 50, 60, 61, in which the stopper elements 50, 60, 61 have an L-shape, and an L-shaped cross section in a plane comprising the adjacency direction 130 and orthogonal to the first skin-facing surface 80. Except for the shape of the stopper elements 50, 60, 61, the sixth movement restraining device 6 is identical to the fifth movement restraining device 5 shown in Figures 7 and 8.

The shape of the first stopper element 50 - and the identical shapes of the second stopper element 60 and of the further stopper elements 61 - is chosen such that the surface normal 210 of the abutment surface 70 is oriented at an angle of about 20° with respect to the surface normal 200 of the first skin-facing surface 80.

In Figure 9, the first stopper element 50 and the second stopper element 60 are not in an abutting pivot position.

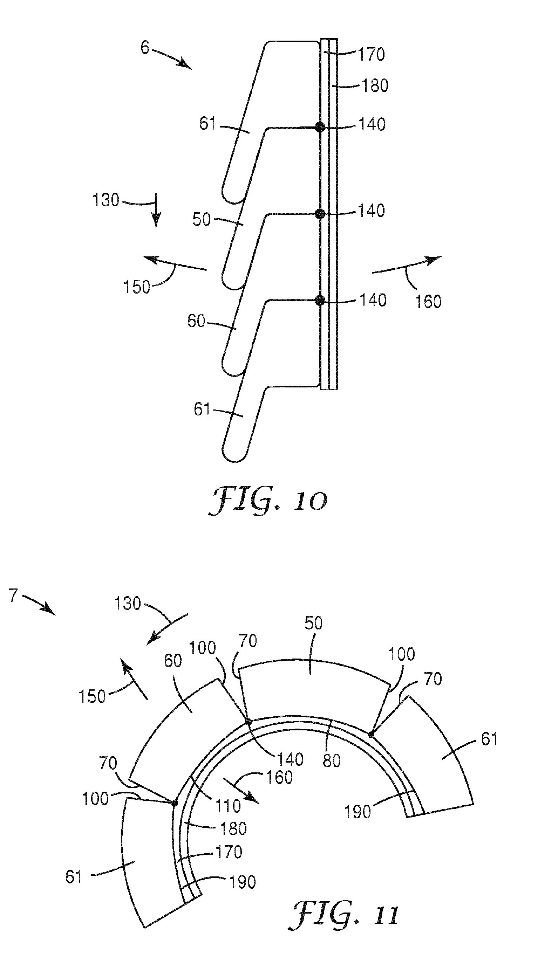

**Figure 10** shows, in side view, the sixth movement restraining device 6 in a state, in which the second stopper element 60 is in an abutting pivot position with respect to the first stopper element 50, and in which all of the stopper elements 50, 60, 61 are in an abutting pivot position relative to an adjacent stopper element 50, 60, 61. In this position, further pivoting of the second stopper element 60 and of any further stopper element 60, 61 in the first pivot direction 150 is impossible, while pivoting in the second pivot direction 160 is not impeded.

The abutting pivot position of the second stopper element 60 is not necessarily a position where the first skin-facing surface 80 and the second skin-facing surface 110 are parallel to each other or lie in the same plane. For scenarios where it is beneficial that the body joint cannot be straightened, the abutting pivot position may be defined such that these skin facing surfaces 80, 110 are tilted with respect to each other. If the skin facing surfaces 80,110 are flat, their surface normals may then form an angle between them. For movement restraining devices that comprise further stopper elements 61 beyond the first and the second stopper elements 50, 60, it may be beneficial if the abutment surfaces 70 and the abutting surfaces 100 of all stopper elements 50, 60, 61 are arranged and shaped such that the entire device defines an abutment for the body joint, when the body joint is in a bent, non-straight rotation position. An example of such a device is shown in the following:

**Figure 11** is an idealized side view of a seventh movement restraining device 7 according to the invention. It is similar to the movement restraining devices 1, 2, 3, 4, 5, 6 shown above, except for the shape of the stopper elements 50, 60, 61. It comprises a first stopper element 50, a second stopper element 60, and two further stopper elements 61, with their skin-facing surfaces 80, 110, 190 affixed on one major surface of a flexible carrier tape 170 carrying an adhesive layer 180 on its opposed major surface. The stopper elements 50, 60, 61 have identical shapes, namely the shape of a segment of a cylinder wall with parallel-curved outer edges. The skin-facing surfaces 80, 110, 190 are curved. The surfaces of the stopper elements 50, 60, 61 are straight, i.e. not curved, in a direction perpendicular to the plane of the drawing.

Figure 11 shows the second stopper element 60 in a pivot position relative to the first stopper element 50 which is not an abutting pivot position. This is evident from the fact that the abutting surface 100 of the second stopper element 60 is not in contact, i.e. surface contact, with the abutment surface 70 of the first stopper element 50. Similarly, the left-hand side further stopper element 61 is in a pivot position relative to the second stopper element 60 which is not an abutting pivot position. Actually, none of the stopper elements 50, 60, 61 is in an abutting pivot position relative to an adjacent stopper element 50, 60, 61.

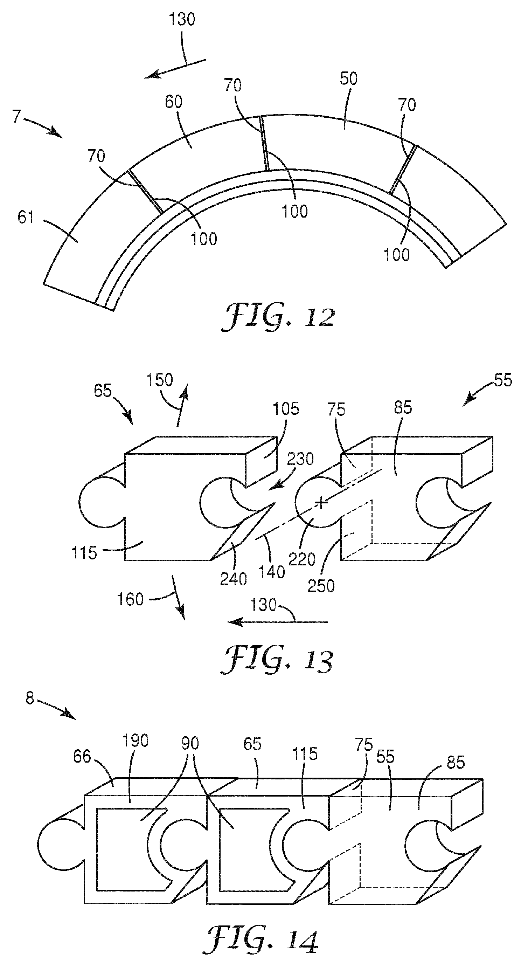

The abutting pivot position of the seventh movement restraining device 7 is shown in **Figure 12****.** The abutting surface 100 of the second stopper element 60 is in contact with the abutment surface 70 of the first stopper element 50. In fact, the entire device 7 provides an abutment for a body joint to which it may be attached, because all pairs of stopper elements 50, 60, 61 and adjacent stopper elements 50, 60, 61 are in an abutting pivot position relative to each other. This is because all abutting surfaces 100 of the stopper elements are in surface contact with the abutment surfaces 70 of the adjacent stopper elements 50, 60, 61.

The overall shape of the seventh movement restraining device 7, when all stopper elements are in an abutting pivot position relative to their adjacent stopper element 50, 60, 61, is a bent shape. As with all previously described movement restraining devices 1, 2, 3, 4, 5, 6, this overall shape defines an abutment position for a body part rotating about a body joint, when the movement restraining device 1, 2, 3, 4, 5, 6, 7 is suitably attached to the body joint.

The pivot axis for the pivoting movement of the second stopper element 60 relative to the first stopper element 50 can be defined by other means than by a carrier tape 170 or by the skin to which the stopper elements are attached. This is described in the following.

**Figure 13** shows, in a perspective sketch, a first stopper element 55 and a second stopper element 65 as they may be comprised in a further movement restraining device. They are shown separately for greater clarity. They can be engaged with each other and with further stopper elements 66 (shown in Figure 14) to form a movement restraining device. They can be engaged by inserting a protrusion 220 of the first stopper element 55 into a recess 230 of the second stopper element 65.

Both stopper elements 55, 65 have identical outer shapes, similar to the shape of a thick jigsaw puzzle piece. The first stopper element 55 comprises a flat abutment surface 75 and a first skin-facing surface 85. The abutment surface 75 is oriented perpendicularly to the first skin-facing surface 85. The first stopper element 55 can be attached to the skin via an adhesive layer (not shown), which is arranged on the first skin-facing surface 85.

The second stopper element 65 comprises an abutting surface 105 and a second skin-facing surface 115. The second stopper element 65 can be attached to the skin via a second adhesive layer (not shown), arranged on the second skin-facing surface 115. When comprised in a movement restraining device, the second stopper element 65 is arranged adjacent to the first stopper element 55. The adjacency direction is indicated by arrow 130.

The second stopper element 65 is pivotable relative to the first stopper element 55, about a virtual pivot axis 140, which extends perpendicularly to the first skin-facing surface 85. The virtual pivot axis 140 is also perpendicular, i.e. orthogonal, to the adjacency direction 130.

When the first stopper element 55 and the second stopper element 65 are engaged with each other, the second stopper element 65 can be pivoted relative to the first stopper element 55 about the pivot axis 140.

The abutting pivot position is the pivot position in which the abutment surface 75 and the abutting surface 105 are in contact with each other. In that abutting pivot position, further pivoting in a first pivot direction 150 is prevented, while pivoting in an opposed, second pivot direction 160 is possible. Due to the shape and arrangement of the stopper elements 55, 65, pivoting in the second pivot direction is possible up to a secondary abutting pivot position in which a secondary abutting surface 240 of the second stopper element 65 abuts against a secondary abutment surface 250 of the first stopper element. The arrangement and shape of the secondary abutting surface 240 and of the secondary abutment surface 250 define the secondary abutting pivot position. A movement restraining device comprising these stopper elements 55, 65 thereby provides two different abutment positions to the body part rotating about a joint axis of a body joint to which this movement restraining device is attached. These abutment positions can be pre-determined by suitably shaping and arranging the abutment surfaces 75, 250 and the abutting surfaces 105, 240.

**Figure 14** is a perspective sketch of an eighth movement restraining device 8 according to the invention, comprising the stopper elements 55, 65 shown in Figure 13 and a further stopper element 66. The device 8 is shown with the second stopper element 65 being in the abutting pivot position relative to the first stopper element 55. A respective adhesive layer 90 is arranged on each of the skin-facing surfaces 85, 115, 190 of the stopper elements 55, 65, 66. These adhesive layers form skin attachment means for attaching the stopper elements 55, 65, 66 to skin at the body joint.

The eighth movement restraining device 8 is particularly suitable for restraining the movement of a human wrist. The device 8 can be attached, via the adhesive layers 90, to the ulna side of the wrist and to the edge, i.e. the outer side, of the hand. When the wrist bends inward, the stopper elements 55, 65, 66 pivot relative to adjacent stopper elements 55, 65, 66 with the edge of the palm. The device 8 can thus prevent inward bending of the palm beyond a certain angle.

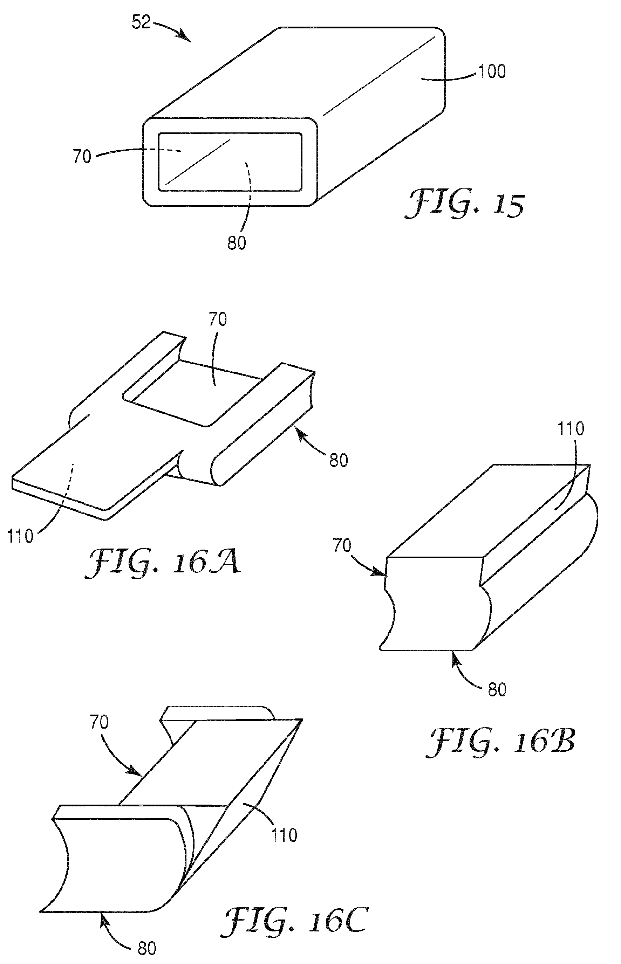

**Figure 15** is a perspective sketch of a stopper element 52 that can be used as first stopper element, second stopper element and/or further stopper element in movement restraining devices like the first movement restraining device 1. Its outer shape is the shape of a brick, however, the stopper element 52 is hollow and a side wall is missing. This may make the stopper element 52 lighter in weight and potentially deformable to a certain degree, so that it can provide for a somehow softer abutment when in the abutting pivot position. Its skin-facing surface 80 is not visible, because it is oriented downwards (in the drawing). The vertical surface oriented towards the left (in the drawing) is the abutment surface 70. The vertical surface towards the right may serve as an abutting surface 100 for an adjacent stopper element.

**Figures 16A, 16B and 16C** are perspective sketches of three further possible shapes of stopper elements that can be used as first stopper element 50, second stopper element 60 or further stopper element 61. They are particularly useful in movement restraining devices in which a plurality of stopper elements have an identical shape, or in which all stopper elements have an identical shape. In the devices shown in Figures 16A, 16B and 16C, the respective skin-facing surface 80 is oriented towards the bottom (in the drawing). Stopper elements like the one shown in Figures 7 and 8, or the one shown in Figure 16A, in which the abutment surface 70 is oriented parallel to the skin-facing surface 80, may result in a particularly flat and low profile of a movement restraining device.

## Claims

1. Movement restraining device (1, 2, 3, 4, 5, 6, 7, 8) for partially immobilizing a body joint (10) facilitating rotation of a body part (20) about a joint axis (40), the restraining device comprising
a) a first stopper element (50, 52, 55), comprising an abutment surface (70, 75);
b) a second stopper element (60, 65),
- comprising an abutting surface (100, 105);
- being arranged, in an adjacency direction (130), adjacent to the first stopper element;
- being pivotable, relative to the first stopper element, about a virtual pivot axis (140); and
c) skin attachment means (90, 120, 170, 180) for attaching the first and the second stopper element to skin at the body joint to be partially immobilized;
wherein the second stopper element can be pivoted into an abutting pivot position, in which position the abutting surface is in surface contact with the abutment surface, the surface contact preventing pivoting in a first pivot direction (150), while allowing pivoting in a second pivot direction (160), opposite to the first pivot direction.

2. Movement restraining device according to claim 1, wherein the first stopper element and/or the second stopper element has the shape of a parallelepiped.

3. Movement restraining device according to any one of the preceding claims, wherein the first stopper element and the second stopper element have identical outer shapes.

4. Movement restraining device according to any one of the preceding claims, wherein the first stopper element comprises a first skin-facing surface (80, 85), wherein the second stopper element comprises a second skin-facing surface (110, 115), and
wherein the skin attachment means is adapted for attaching the first and the second stopper element to skin in a way, that the first and the second skin-facing surfaces are oriented towards the skin.

5. Movement restraining device according to claim 4, wherein the abutment surface is flat and is oriented perpendicularly to the first skin-facing surface.

6. Movement restraining device according to claim 4 or claim 5, wherein the abutment surface is flat, and wherein the surface normal (210) of the abutment surface is oriented at an angle of between 60° and 120° with respect to the surface normal (200) of the first skin-facing surface.

7. Movement restraining device according to claim 4, wherein the abutment surface is flat and is oriented parallel to the first skin-facing surface.

8. Movement restraining device according to any one of claims 4 to 7, wherein the pivot axis is oriented parallel to the first skin-facing surface.

9. Movement restraining device according to any one of claims 4 to 7, wherein the pivot axis is oriented perpendicular to the first skin-facing surface.

10. Movement restraining device according to any one of preceding claims, wherein the skin attachment means comprises a layer of adhesive (180) and a carrier tape (170) having two opposed major surfaces, and wherein the layer of adhesive (180) is arranged on one of the major surfaces of the carrier tape.

11. Movement restraining device according to any one of the preceding claims, wherein the first stopper element and the second stopper element are attached to each other by the skin attachment means.

12. Movement restraining device (2, 3, 4, 5, 6, 7, 8) according to any one of the preceding claims, comprising one or more further stopper elements (61, 66), each comprising an abutment surface (70, 75), and an abutting surface (100, 105), each of the further stopper elements being arranged, in an adjacency direction (130), adjacent to the first stopper element, the second stopper element or one of the further stopper elements,
wherein each further stopper element is pivotable, relative to the adjacent stopper element, about a virtual pivot axis (140),
wherein the movement restraining device further comprises skin attachment means (90) for attaching the further stopper elements to skin at the body joint to be partially immobilized; and
wherein the abutment surface (70, 75) of each further stopper element is arranged and/or shaped such that, in an abutting pivot position, the abutment surface of the further stopper element is in contact with the abutting surface (100, 105) of the adjacent stopper element, thereby preventing pivoting in a first pivot direction, while allowing pivoting in a second pivot direction, opposite to the first pivot direction.

13. Movement restraining device according to any one of the preceding claims,
wherein the first stopper element or the second stopper element or a further stopper element comprises a tube organizer for routing and/or fixation of catheter tubes or lumen.

14. Movement restraining device according to any one of the preceding claims, further comprising a first electrical contact element and a second electrical contact element, at least one of which is connectable to an indicator device for providing a visual, audible or electronic indication when the contact elements are in electrical contact with each other,
wherein the first contact element can be brought into electrical contact with the second contact element by pivoting the second stopper element into the abutting pivot position.

15. Method of partially immobilizing a body joint (10) facilitating rotation of a body part (20) about a body joint axis (40), the method comprising the steps of
a) providing a movement restraining device (1, 2, 3, 4, 5, 6, 7, 8) according to claim 1; and
b) attaching the first stopper element (50, 52, 55) and the second stopper element (60, 65) to skin at the body joint such that the pivot axis (140) is oriented parallel to the body joint axis (40).
